# EUROPEAN PATENT SPECIFICATION

(11) **EP 2 948 048 B1**
(45) Date of publication and mention of the grant of the patent: **30.11.2016**
(21) Application number: 14700544.1
(22) Date of filing: 15.01.2014
(51) Int. Cl.: A61B 5/01, A61B 5/053

(54) **CONTINUOUS NON-IVASIVE MEASUREMENT OF TISSUE TEMPERATURES BASED ON IMPEDANCE MEASUREMENTS**
KONTINUIERLICHE NICHT INVASIVE MESSUNG VON GEWEBETEMPERATUREN AUF BASIS VON IMPEDANZMESSUNGEN
MESURE NON INVASIVE CONTINUE DE TEMPÉRATURES DE TISSU BASÉE SUR DES MESURES D'IMPÉDANCE

(30) Priority: 22.01.2013 DE 102013000966; 23.01.2013 US 201361755626 P
(43) Date of publication of application: 02.12.2015
(73) Proprietor: Zimmer MedizinSysteme GmbH, 89231 Neu-Ulm (DE)
(72) Inventor: ZIMMER, Armin, 89233 Neu-Ulm (DE)
(74) Representative: Sawodny, Michael-Wolfgang
(86) International application number: PCT/EP2014/000084
(87) International publication number: WO 2014/114433

(56) References cited:
- JP-A- 2004 254 995
- US-A1- 2003 179 808
- US-A1- 2008 302 675
- US-B1- 6 236 886

## Description

The invention relates to a method for direct continuous non-invasive measurement of temperatures in a tissue, preferably at different depths as well as an apparatus, in particular for carrying out such a method.

Hitherto in medicine, if one wished to measure the body internal temperature, this was usually accomplished invasively, i.e. using a measuring needle or a sensor inserted under the skin. A disadvantage of this method in addition to a certain risk of infection was that this was associated with not inconsiderable pain.

A medical measuring apparatus has become known from EP 2 096 989 B1 which determines the bioelectric impedance locally with electrodes. In EP 2 096 989 B1 the cardiac frequency and the pulse amplitude are determined with the aid of the bioelectric impedance. In turn, according to EP 2 096 989 B1 the body temperature can be determined from the pulse amplitude. A disadvantage with the measuring apparatus according to EP 2 096 989 B1 is that the body temperature cannot be derived directly but on the contrary a derivation from blood values is always necessary.

US 6,236 886 B1 discloses a method for the continuous, non-invasive measurement of temperatures in a tissue. According to US 6,236,886 B1 changes in the temperature of internal organs can be detected because temperature changes are accompanied by changes in conductivity of the tissue.

US 2003/179,808 A1 shows a non-invasive apparatus and method for measuring a temperature of a portion of a living body including a signal receiving unit receiving electromagnetic wave signals, emitted from the portion of a living body to be measured.

Either document US 6,236,886 B1 or US 2003/179,808 A1 could be determined as closest state of the art.

It is therefore the object of the invention to provide a method and an apparatus which overcomes the disadvantages of the prior art and in particular enables the most exact possible determination of the temperature in simple and reproducible manner.

According to the invention, this object is solved by a method for the continuous non-invasive measurement of temperatures in a tissue, where a current its guided into the tissue by means of at least one feed electrode and a voltage caused by the current in the tissue is measured by means of at least one measuring electrode and from this the resistance or the impedance of the magnitude of the impedance of the tissue through which the current flows is determined. The invention is characterized in that the current is a frequency-variable alternating current and the magnitude of an averaged impedance over a frequency range, wherein the frequency range is from 330 kHz to 950 kHz.

The method described utilizes the fact that the resistance or the impedance or the magnitude of the impedance of the tissue increases or decreases with temperature. If a current is applied, the voltage correlates with the tissue temperature and the resistance or the impedance or the magnitude of the impedance determined from these quantities is a direct measure for the temperature prevailing in the tissue part.

The direct determination of the temperature of the tissue from the impedance measurement without a determination of blood values, as described for example, in EP 2 096 989 B1, has the advantage that even in tissue parts with poor circulation it is possible to determine the body temperature.

Preferably in the method according to the invention, in order to avoid galvanic effects an alternating current is fed as current into the tissue via the feed electrodes having a current density in the range of a few microamperes to a few milliamperes. The potential field formed in the tissue is then primarily dependent on the structure and temperature of the tissue.

The voltage formed in the tissue as a result of the potential field can be measured, for example using measuring electrodes. The specific resistance or the impedance or the magnitude of the impedance of the tissue through which current flows can then be calculated from the supplied current and the measured voltage taking into account the measurement geometry of the electrodes, where the specific resistance is the reciprocal of the specific conductance of the tissue.

For the specific resistance it holds that pₛ = K * U / I, where U is the measured voltage, I is the supplied current and K is a geometrical factor which is dependent on the electrode arrangement of the measuring electrodes and the feed electrodes. The feed electrodes and the measuring electrodes can be provided in various arrangements, for example
- a Wenner arrangement
- a Schlumberger arrangement
- a three-point system,
- a double dipole system
- a Lee arrangement

In the arrangement of feed and measuring electrodes which preferably consists of a non-metallic material, a Wenner arrangement is preferred.

In order to determine the temperature or tissue temperature, a predefined frequency range, in particular from 330 kHz to 1000 kHz is covered with the aid of a phase locked loop (PLL), an impedance curve is recorded and at the same time as this, the variation of the resistance and resulting curve slopes are calculated. Mathematical methods can also be used to be able to determine the tissue temperature

In order to be able to determine the temperature in the tissue depth, in a further developed embodiment it can be provided to provide at least two feed electrodes and/or at least two measuring electrodes which have a distance from one another. If the distance is varied, the current penetrates to varying depth into the tissue. Values for the resistance, the impedance or the magnitude of the impedance can again be determined from the determined voltage for various distances. These values of the resistances, the impedances or the magnitude of the impedance can represent the temperatures in variously deep tissues.

If the absolute value for the temperature is to be determined from the determination of the resistance, the impedance or the magnitude of the impedance, it is advantageous to determine a reference temperature independently by means of an independent measurement method e.g. a sensor device such as a skin sensor and/or an IR thermometer. A resistance determined by means of the method according to the invention, an impedance or a magnitude of an impedance or an impedance profile can be assigned to the independently determined reference temperature. Thus, a certain impedance value correlates with a certain temperature value. This enables the assignment of an absolute temperature.

Preferably an alternating current is supplied as current. However, a direct current or a pulsed direct current would also be possible. In order to be able to measure a frequency-dependent impedance, the applied current is frequency-variable.

By means of the reference temperature, the resistance, the impedance or the magnitude of the impedance or the impedance profile can be standardized or calibrated in relation to the temperature or a zero setting can be performed.

If a frequency-variable current is supplied, the frequency range over which the current is varied and the impedance or the magnitude of the impedance is determined in a frequency-dependent manner is a range from a few Hz to several hundred MHz, in particular the range from 10 kHz to 1000 kHz, preferably from 300 kHz to 1000 kHz.

In addition to the method, the invention also provides an apparatus for non-invasive measurement of the tissue temperature. The apparatus comprises at least one feed electrode for supplying a current into a tissue and at least one measuring electrode. The feed electrodes can consist of metallic or also of non-metallic materials, for example, it would be possible to make the feed electrodes from stainless steel. In addition to the feed electrodes for supplying the current, the apparatus comprises at least one measuring electrode which can also be fabricated from a metallic or non-metallic material. The measuring electrodes can be AgAgCl multiple electrodes in the form, for example, of Ag-AgCl plates or Ag-AgCl single electrodes preferably as adhesive electrodes. The AgAgCl electrodes are not suitable as feed electrodes as a result of their chemical propertied.

In addition to the feed electrodes and the measuring electrodes, the apparatus further comprises a unit with the aid of which it is possible to determine the resistance or the impedance or the magnitude of the impedance or the impedance profile in the tissue through which current flows from the supplied current and the measured voltage and then determine the temperature in the tissue from the determined resistance or the determined impedances or the determined impedance profile over the frequency. To this end, various impedance values are determined which are measured by frequency variability relating to their impedance.

The apparatus according to the invention comprises a frequency-variable generator by which means the current is supplied. A possible generator would be a tuneable sine generator having a frequency range of 300 kHz to 1000 kHz, which delivers constant current in the range of a few µA to a few mA. The recording of the measured values is then made in the mV range, i.e. at a voltage of <100 mV. It is particularly preferable if the frequency-variable generator is based on a microcontroller circuit. In one embodiment of the invention it can be provided that the tuneable generator is in particular a generator which is tuneable by means of a phase-locked loop (PLL).

In addition to a sine signal having one frequency, other periodic signals are also feasible such as, for example, a rectangular or triangular signal which can also be varied in their frequency. Curve shapes other than sine signals for the feed signal have the advantage that other harmonics can be specifically set. In addition to periodic signals, monophase current pulses are also possible.

As described previously, a sensor device can be provided for zero setting or calibration. With the aid of the sensor device it is possible to determine an absolute temperature independently of the impedance measurement, e.g. the surface temperature of the skin. This absolute value can then again be assigned an impedance which is present at the absolute temperature so that impedance values correlated with the temperature, Since a non-invasive method is involved here, surface sensors which can be applied to the skin are provided for this purpose.

The invention will be described in more detail hereinafter with reference to exemplary embodiments.

In the figures:
- Fig. 1a-1b: shows the measurement principle of the invention
- Fig. 2: shows the Wenner electrode arrangement
- Fig. 3a: shows a family of characteristics for the apparatus according to the invention which shows the profile of the impedance value Z [Ω] for a measurement example to determine the temperature as a function of the frequency f [Hz] of the supplied alternating current for various temperatures.
- Fig. 3b.: shows the temperature dependence of the impedance
- Fig. 4: shows the structure of an apparatus according to the invention

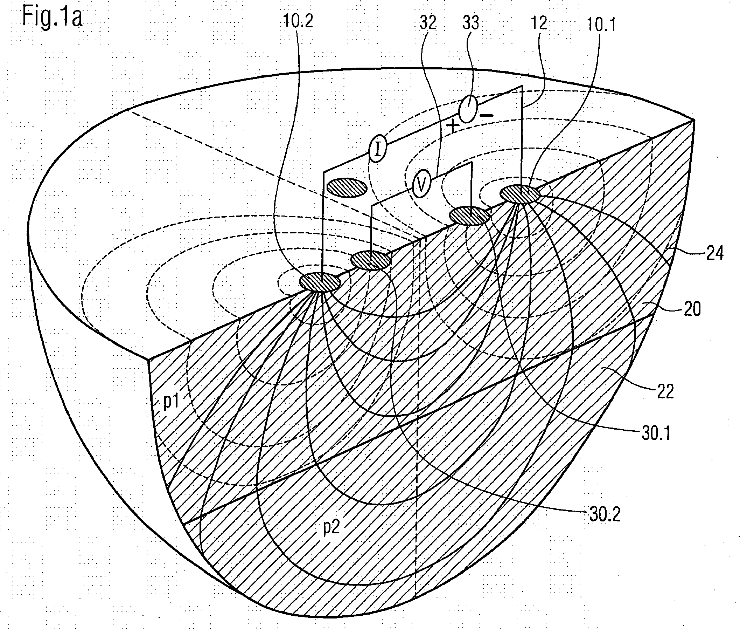

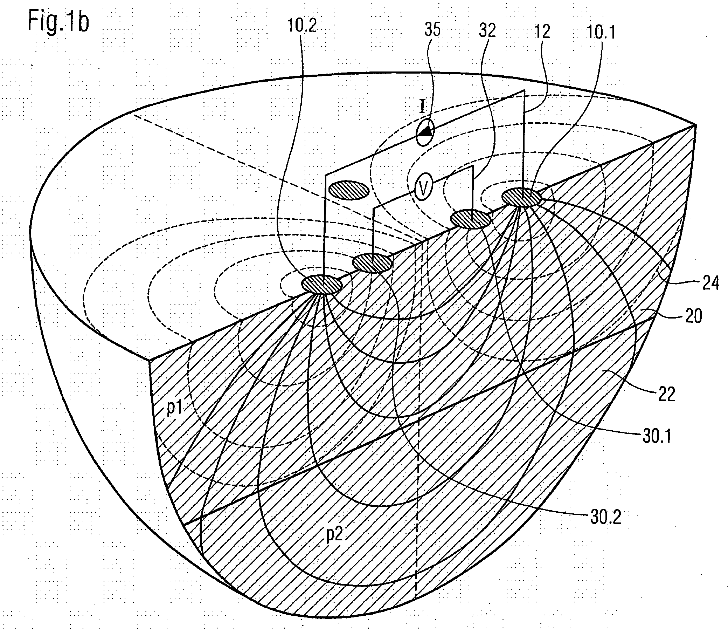

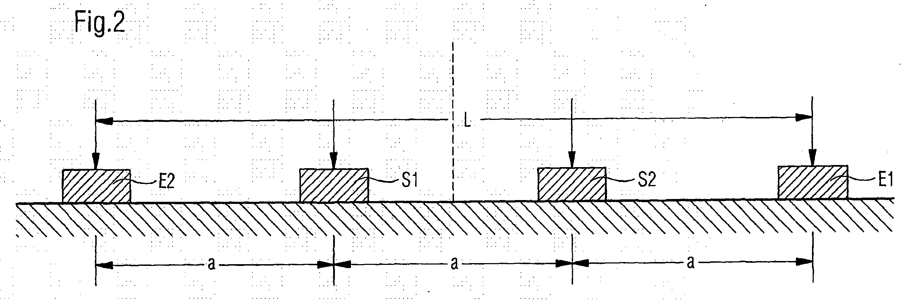

Figures 1a and 1b show the measurement principle of the method. The electrodes are, without being restricted to this, as shown in Figure 2, arranged according to Wenner, i.e. two feed electrodes, 10.1, 10.2 are provided by which means a current 12 is fed into a tissue 20 lying below the electrodes. The current lines caused by the current inside the tissue are characterized by reference number 22. As a result of the current flow through the tissue 20, a potential field with potential lines 24 is formed and a voltage 32 is determined with the aid of the measuring electrodes 30.1, 30.2. From the measured voltage, knowing the supplied current, the resistance or the impedance or the magnitude of the impedance or an impedance value can be determined which is a direct measure for the temperature in the tissue, as shown hereinafter.

Figure 1 a shows the supply of current with the aid of a voltage source 33. Alternatively Figure 1 b shows the supply with a current source 35. The same components as in Figure 1 a are characterized by the same reference numbers.

Figure 2 shows
a special Wenner electrode arrangement - without being restricted to this. In the Wenner electrode arrangement, the feed electrodes E1, E2 should be equated to the electrodes 10.1, 10.2 shown in Figure 1a, the measuring electrodes S1, S2 are designated by 30.1 and 30.2 in Figure 1a. The distance between the feed electrodes E1, E2 is specified by L, the distance between feed electrode E1 and measuring electrode S1 as well as measuring electrodes S1 and S2 and measuring electrode S2 and feed electrode E2 is always equidistant as a. The geometric factor K for the Wenner arrangement is then obtained as K = a. Although a Wenner electrode arrangement is shown here, other electrode arrangements are also feasible, such as a Schlunlberger arrangement, a three-point system, a double dipole system or a Lee arrangement. The electrodes substantially differ by the electrode placement and the geometric factor K.

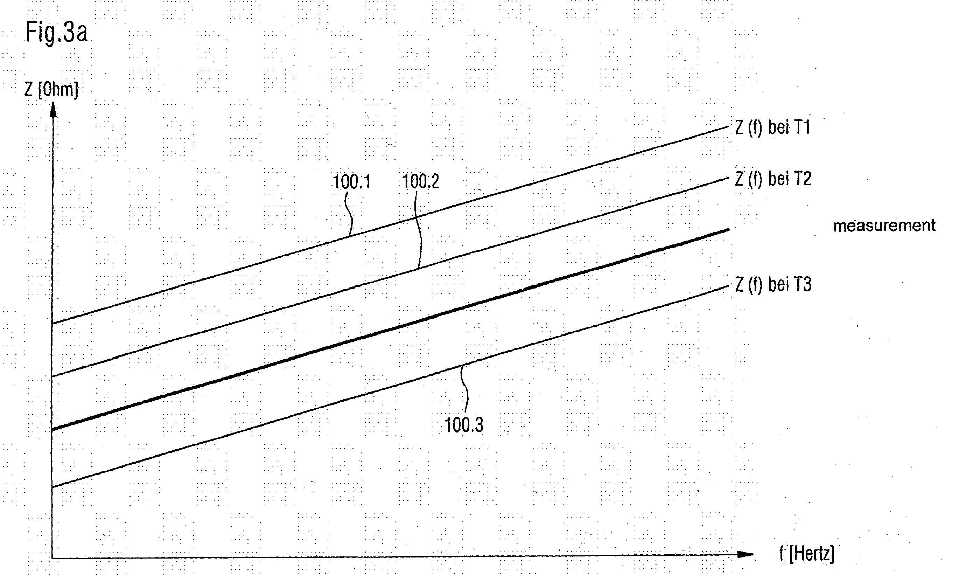

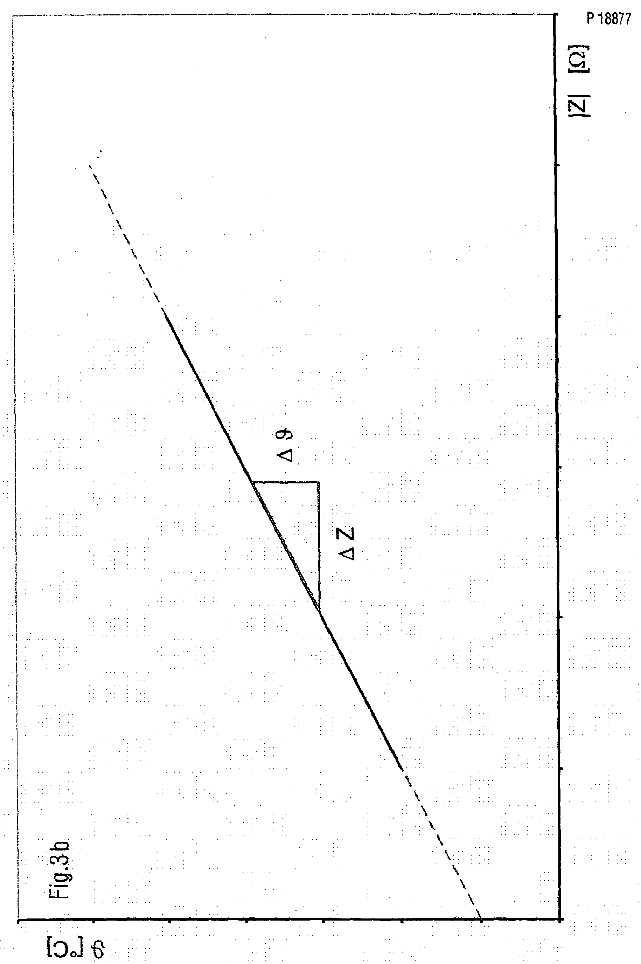

Figure 3a shows a family of characteristics plotted for the apparatus according to the invention which shows the behaviour of the impedance value [Ω] as a function of the frequency f [Hz] of the supplied alternating current for various temperatures. As can be seen from Figure 3, for each temperature T1, T2, T3, a specific characteristic 100.1, 100.2, 100.3 is determined as a function of the frequency for the impedance As shown in Figure 3, a family of characteristics of the frequency-dependent impedance Z[Ω] is thus obtained depending on the body temperature T in Kelvin. The behaviour Z[Ω] of the tissue impedance in the frequency range between 330 kHz and 950 kHz is linear with respect to the tissue temperature to be measured, i.e. the lines for the temperatures T1, T2 and T3 are displaced parallel to one another and the distance of the lines for different temperatures is equidistant for different frequencies.

In the specified frequency range of 330 kHz to 950 kHz, as shown in Figure 3b, this has the result that a linear relationship is found between temperature and impedance in the specified frequency range. Figure 3b shows this linear relationship. Naturally as a result of the equidistance of the curves in Figure 3a the linear relationship between temperature and impedance also applies when the impedance is determined for an averaged frequency in the specified frequency range. The determination of an impedance over an averaged frequency range e.g. from 330 kHz to 950kHz is advantageous since a subsequent averaging will improve the result in most cases. If the body temperature at the observed location changes, e.g. due to supply of heat, for example, during a heat treatment of the tissue, the tissue temperature and thus the impedance for a certain frequency or a certain frequency range or an averaged frequency increase. As a result of the linearity of impedance Z[Ω] and temperature ϑ [°C], as shown in Figure 3b, the temperature rise Δϑ can be determined from the increase in impedance ΔZ.

As a result of this behaviour, it is possible to carry out non-invasive temperature measurements by the indirect route of the tissue impedance in the range of 330 kHz to 950 kHz. The family, of characteristics is determined whereby, for example, a tissue surface or a skin sample is heated to different temperatures by means of a heating device, for example, a heat lamp. In order to be able to assign absolute temperatures to the impedance values, reference measurements can be carried out. A possible reference temperature can be the surface temperature or the ear temperature of the patient. The surface temperature can be measured, for example, with the aid of a surface sensor as reference.

If a special measurement is carried out, knowing the family of characteristics as described for Figure 3a and Figure 3b, the temperature can be determined. The straight line shown in Figure 3b which gives the relationship of impedance and temperature is a direct measure for the temperature. The variable frequency substantially increases the measurement accuracy of the method since an averaged impedance value for a frequency range can be assigned to a temperature value. This averaged impedance value also varies linearly with temperature so that a temperature rise in the tissue can be detected directly through an increase in the impedance value. It is particularly preferred if the parameter field shown in Figure 3a is a standardized Z-f parameter field which can be used for all measurements. The determination of temperature is then confined to reading off values.

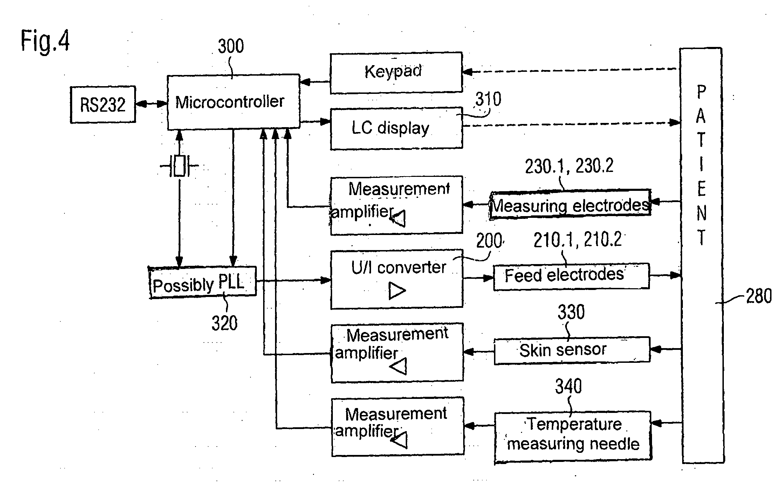

A schematic diagram of the apparatus for determining the tissue temperature is described in Figure 4.

The apparatus for use with the invention comprises on the one hand a U-I converter 200, by which means a current, preferably a constant current, is applied to the tissue of a patient 280 via the feed electrodes 210.1, 210.2. The current acting on the tissue of the patient 280 leads to the formation of a potential field and thus to a voltage which can be determined by means of the measuring electrodes 230.1, 230.2. The voltage received by the measuring electrodes 230.1, 230.2 is amplified by the measuring amplifier and supplied to a microcontroller 300. In the microcontroller the frequency-dependent impedance is evaluated and calculated from the applied current and the measured voltage. The impedance is in turn displayed on an LCD display 310 as a function of the frequency. The microcontroller 300 further controls the frequency-variable generator 320, which can be configured as a PLL generator and whose signal is converted via the U-I converter 200 into a current with constant amplitude which is supplied to the patient via the feed electrodes 210.1, 210.2. For standardization and calibration purposes it can be provided that, in addition to the electronic and computational determination of the tissue temperature by means of the measured voltage and the supplied current intensity, a reference measurement is made, for example, with the aid of a skin sensor 330 or a temperature measuring needle 340, which can record a depth-dependent temperature in the tissue. The skin sensor 330 is a special type of surface sensor whereas the temperature measuring needle enables a depth-dependent measurement. Both measurement methods can be used for calibration and/or standardization purposes.
According to the invention, an averaged impedance value for the frequency range from 330 kHz to 950 kHz is assigned to a temperature value.

With the apparatus according to the invention, an apparatus and a method are provided for the first time which allow the body temperature of a proband to be determined non-invasively directly in a very simple manner by means of a simple impedance measurement. The method and the apparatus are suitable for all areas of temperature acquisition such as long-term recordings or bedside monitoring or monitoring. Furthermore it can be used in intensive care, in operating and anaesthesia operation and in tumour therapy and in particular for monitoring temperature in therapies or applications in which heat or cold is applied to patients.

## Claims

1. Method for the continuous, non-invasive measurement of temperatures in a tissue, wherein a current is supplied to the tissue (20) by means of at least one feed electrode (10.1, 10.2) and a voltage (U) caused by the current (I) is measured by means of at least one measuring electrode (30.1, 30.2) and from this a magnitude of an impedance of the tissue (20) through which the current flows is determined,
**characterized in that**
the current is a frequency-variable alternating current and a tissue temperature in the tissue is determined from the magnitude of an averaged impedance over frequency range, wherein the frequency range is from 330 kHz to 950 kHz.

2. The method according to claim 1,
**characterized in that** a reference temperature is determined by
means of a measurement method and a certain resistance or a certain magnitude of the impedance is assigned.

3. The method according to one of claims 1 to 2,
**characterized in that**
the measurement method performs the determination of the reference temperature with the aid of a sensor device, in particular a skin sensor (330) and/or an IR thermometer.

4. The method according to one of claims 1 to 3,
**characterized in that** the method is carried out using at least two feed
electrodes, a first feed electrode and a second feed electrode, wherein first and second feed electrode have a first distance from one another and/or using at least two measuring electrodes a first measuring electrode and a second measuring electrode, wherein first and second measuring electrode have a second distance from one another.

5. The method according to claim 4,
**characterized in that** the first distance between the first and second feed electrode and/or the second distance between the first and second measuring electrode is varied in order to determine the tissue temperature at various depths.

6. The method according to one of claims 1 to 5,
**characterized in that** a current in the frequency range is supplied by means of a frequency-variable generator (320).

7. Apparatus for continuous non-invasive measurement of temperatures in a tissue (20) comprising
- at least one feed electrode (10.1, 10.2) for feeding a current into a tissue;
- at least one measuring electrode (30.1, 30.2) for measuring
the voltage produced by the current in the tissue,
**characterized in that**
- the apparatus comprises a unit for determining the magnitude of
the impedance of the tissue (20) through which current flows and the tissue temperature in the tissue from an averaged impedance over a frequency range from 330 kHz to 950 kHz, wherein the apparatus comprises a frequency-variable generator (320),
in particular based on a microcontroller (300), adapted to provide
a current in the predefined frequency range from 330 kHz to 950 kHz.

8. The apparatus according to claim 7,
**characterized in that**
the apparatus comprises a device for determining a reference temperature which is assigned a certain resistance or a certain magnitude of the impedance.

9. The apparatus according to claim 8,
**characterized in that**
the device for determining the reference temperature is a sensor device, in particular a skin sensor (330)
and/or an IR thermometer.

10. The apparatus according to one of the claims 7 to 9,
**characterized in that**
the frequency-variable generator is a tuneable generator.

11. The apparatus according to one of claims 7 to 10,
**characterized in that**
the frequency-variable generator is adapted to provide a monophase current of
an alternating current having different signal shapes, in particular a
rectangular shape, a triangular shape or a sine shape.

12. Use of a method according to one of claims 1 to 6 for at least one of the following purposes:
- for long-term recording
- for monitoring or bed-side monitoring
- for intensive care
- for monitoring the temperature or temperature behaviour.

13. Use of an apparatus according to one of the claims 7 to 11 for at least one of the following purposes:
- for long-term recording
- for monitoring or bed-side monitoring
- for intensive care
- for monitoring the temperature or temperature behaviour.

## Patentansprüche

1. Verfahren zur kontinuierlichen, nicht invasiven Messung von Temperaturen in einem Gewebe, wobei ein Strom mittels wenigstens einer Speiseelektrode (10.1, 10.2) dem Gewebe (20) zugeführt wird und eine durch den Strom (I) verursachte Spannung (U) mittels wenigstens einer Messelektrode (30.1, 30.2) gemessen wird und hieraus ein Betrag einer Impedanz des vom Strom durchflossenen Gewebes (20) bestimmt wird, **dadurch gekennzeichnet, dass**
der Strom ein frequenzvariabler Wechselstrom ist und eine Gewebetemperatur in dem Gewebe aus dem Betrag einer über einen Frequenzbereich gemittelten Impedanz bestimmt wird, wobei der Frequenzbereich von 300 kHz bis 950 kHz reicht.

2. Verfahren nach Anspruch 1,
**dadurch gekennzeichnet, dass**
eine Bezugstemperatur mittels eines Messverfahrens bestimmt wird und ein gewisser Widerstand oder ein gewisser Betrag der Impedanz zugewiesen wird.

3. Verfahren nach einem der Ansprüche 1 bis 2,
**dadurch gekennzeichnet, dass**
das Messverfahren die Bestimmung der Bezugstemperatur mit Hilfe einer Sensoreinrichtung, insbesondere eines Hautsensors (330) und/oder eines IR-Thermometers, durchführt.

4. Verfahren nach einem der Ansprüche 1 bis 3,
**dadurch gekennzeichnet, dass**
das Verfahren durchgeführt wird, indem wenigstens zwei Speiseelektroden, eine erste Speiseelektrode und eine zweite Speiseelektrode, verwendet werden, wobei die erste und zweite Speiseelektrode einen ersten Abstand zueinander aufweisen, und/oder indem wenigstens zwei Messelektroden, eine erste Messelektrode und eine zweite Messelektrode, verwendet werden, wobei die erste und zweite Messelektrode einen zweiten Abstand zueinander aufweisen.

5. Verfahren nach Anspruch 4,
**dadurch gekennzeichnet, dass**
der erste Abstand zwischen der ersten und zweiten Speiseelektrode und/oder der zweite Abstand zwischen der ersten und zweiten Messelektrode variiert wird, um die Gewebetemperatur in unterschiedlichen Tiefen zu bestimmen.

6. Verfahren nach einem der Ansprüche 1 bis 5,
**dadurch gekennzeichnet, dass**
mittels eines frequenzvariablen Generators (320) ein Strom in dem Frequenzbereich zugeführt wird.

7. Vorrichtung zur kontinuierlichen, nicht invasiven Messung von Temperaturen in einem Gewebe (20), umfassend
- wenigstens eine Speiseelektrode (10.1, 10.2) zum Einspeisen eines Stroms in ein Gewebe;
- wenigstens eine Messelektrode (30.1, 30.2) zum Messen der durch den Strom im Gewebe erzeugten Spannung,
**dadurch gekennzeichnet, dass**
- die Vorrichtung eine Einheit zur Bestimmung des Betrags der Impedanz des Gewebes (20), durch das der Strom fließt, und der Gewebetemperatur in dem Gewebe aus einer über einen Frequenzbereich von 300 kHz bis 950 kHz gemittelten Impedanz umfasst, wobei die Vorrichtung einen frequenzvariablen Generator (320) umfasst, der insbesondere auf einem Mikrocontroller (300) basiert, der geeignet ist, einen Strom in dem vordefinierten Frequenzbereich von 300 kHz bis 950 kHz bereitzustellen.

8. Vorrichtung nach Anspruch 7,
**dadurch gekennzeichnet, dass**
die Vorrichtung eine Einrichtung zur Bestimmung einer Bezugstemperatur umfasst, der ein bestimmter Widerstand oder ein bestimmter Betrag der Impedanz zugewiesen ist.

9. Vorrichtung nach Anspruch 8,
**dadurch gekennzeichnet, dass**
die Einrichtung zur Bestimmung der Bezugstemperatur eine Sensoreinrichtung, insbesondere ein Hautsensor (330) und/oder ein IR-Thermometer ist.

10. Vorrichtung nach einem der Ansprüche 7 bis 9,
**dadurch gekennzeichnet, dass**
der frequenzvariable Generator ein abstimmbarer Generator ist.

11. Vorrichtung nach einem der Ansprüche 7 bis 10,
**dadurch gekennzeichnet, dass**
der frequenzvariable Generator dafür geeignet ist, einen einphasigen Strom oder einen Wechselstrom mit unterschiedlichen Signalformen, insbesondere einer Rechteckform, einer Dreiecksform oder einer Sinusform, bereitzustellen.

12. Verwendung eines Verfahrens nach einem der Ansprüche 1 bis 6 für wenigstens einen der nachfolgenden Zwecke:
- zur Langzeitaufzeichnung,
- zur Überwachung oder bettseitigen Überwachung,
- zur Intensivpflege,
- zur Überwachung der Temperatur oder des Temperaturverlaufes.

13. Verwendung einer Vorrichtung nach einem der Ansprüche 7 bis 11 für wenigstens einen der nachfolgenden Zwecke:
- zur Langzeitaufzeichnung,
- zur Überwachung oder bettseitigen Überwachung,
- zur Intensivpflege,
- zur Überwachung der Temperatur oder des Temperaturverlaufes.

## Revendications

1. Une méthode pour la mesure non-invasive continue des températures dans un tissu, où un courant est fourni au tissu (20) au moyen d'au moins une électrode d'alimentation (10.1, 10.2) et une tension (U) causée par le courant (I) est mesurée au moyen d'au moins une électrode de mesure (30.1, 30.2) et à partir de cela, une magnitude d'une impédance du tissu (20) au travers de laquelle les flux de courant sont déterminés,
**Caractérisée en ce que**
Le courant est un courant alternatif à fréquence variable et une température de tissu dans le tissu est déterminée à partir de la magnitude d'une impédance moyenne sur une gamme de fréquence, où la gamme de fréquence va de 330 kHz à 950 kHZ.

2. La méthode selon la revendication 1,
**Caractérisée en ce qu'**une température de référence est déterminée au moyen d'une méthode de mesure et une certaine résistance ou une certaine magnitude de l'impédance est attribuée.

3. La méthode selon l'une des revendications 1 à 2,
**Caractérisée en ce que**
La méthode de mesure effectue la détermination de la température de référence à l'aide d'un dispositif capteur, en particulier un capteur cutané (330) et/ou un thermomètre IR.

4. La méthode selon l'une des revendications 1 à 3,
**Caractérisée en ce que** la méthode est réalisée à l'aide d'au moins deux électrodes d'alimentation, une première électrode d'alimentation et une deuxième électrode d'alimentation, où la première et la deuxième électrode d'alimentation ont une première distance à partir l'une de l'autre et/ou à l'aide d'au moins deux électrodes de mesure, une première électrode de mesure et une deuxième électrode de mesure, où la première et la deuxième électrodes de mesure ont une deuxième distance à partir l'une de l'autre.

5. La méthode selon la revendication 4,
**Caractérisée en ce que** la première distance entre la première et la deuxième électrode d'alimentation et/ou la deuxième distance entre la première et la deuxième électrode de mesure varie afin de déterminer la température du tissu à plusieurs profondeurs.

6. La méthode selon l'une des revendications 1 à 5,
**Caractérisée en ce qu'**un courant dans la gamme de fréquence est fourni au moyen d'un générateur à fréquence variable (320).

7. Un appareil pour une mesure non invasive continue des températures dans un tissu (20) comprenant
- au moins une électrode d'alimentation (10.1, 10.2) pour apporter un courant dans un tissu ;
- au moins une électrode de mesure (30.1, 30.2) pour mesurer la tension produite par le courant dans le tissu,
**Caractérisé en ce que**
- l'appareil comprend une unité pour déterminer la magnitude de l'impédance du tissu (20) au travers de laquelle le courant circule et la température du tissu dans le tissu à partir d'une impédance moyenne sur une gamme de fréquence de 330 kHz à 950 kHz, où l'appareil comprend un générateur à fréquence variable (320),
En particulier basé sur un microcontrôleur (300) adapté pour fournir un courant dans la gamme de fréquence prédéfinie de 330 kHz à 950 kHz.

8. L'appareil selon la revendication 7,
**Caractérisé en ce que**
L'appareil comprend un dispositif pour déterminer une température de référence à qui est attribuée une certaine résistance ou une certaine magnitude de l'impédance.

9. L'appareil selon la revendication 8,
**Caractérisé en ce que**
Le dispositif pour déterminer la température de référence est un dispositif capteur, en particulier un capteur cutané (330)
Et / ou un thermomètre IR.

10. L'appareil selon l'une des revendications 7 à 9,
**Caractérisé en ce que**
Le générateur à fréquence variable est un générateur réglable.

11. L'appareil selon l'une des revendications 7 à 10,
**Caractérisé en ce que**
Le générateur à fréquence variable est adapté pour fournir un courant monophasé ou un courant alternatif ayant différentes formes de signal, en particulier une forme rectangulaire, une forme triangulaire ou une forme de sinus.

12. Une utilisation d'une méthode selon l'une des revendications 1 à 6 pour au moins un des buts suivants :
- pour un enregistrement à long terme
- pour une surveillance ou une surveillance au chevet du patient
- pour des soins intensifs
- pour une surveillance de la température ou du comportement de la température.

13. Une utilisation d'un appareil selon l'une des revendications 7 à 11 pour au moins un des buts suivants :
- pour un enregistrement à long terme
- pour une surveillance ou une surveillance au chevet du patient
- pour des soins intensifs
- pour une surveillance de la température ou du comportement de la température.
